# EUROPÄISCHE PATENTSCHRIFT

(11) **EP 0 759 997 B1**
(45) Veröffentlichungstag und Bekanntmachung des Hinweises auf die Patenterteilung: **11.06.2003**
(21) Anmeldenummer: 95920818.2
(22) Anmeldetag: 17.05.1995
(51) Int. Cl.: C12N 15/63, C12N 15/70, C12P 21/00

(54) **VERWENDUNG DES TETRACYCLINPROMOTORS ZUR STRINGENT REGULIERTEN PRODUKTION VON REKOMBINANTEN PROTEINEN IN PROKARYONTISCHEN ZELLEN**
USE OF TETRACYCLINE PROMOTER FOR THE STRICTLY REGULATED PRODUCTION OF RECOMBINANT PROTEINS IN PROKARYOTIC CELLS
UTILISATION DU PROMOTEUR DE LA TETRACYCLINE POUR LA PRODUCTION STRICTEMENT REGULEE DE PROTEINES RECOMBINEES DANS DES CELLULES PROCARYOTES

(30) Priorität: 19.05.1994 DE 4417598
(43) Veröffentlichungstag der Anmeldung: 05.03.1997
(73) Patentinhaber: Max-Planck-Gesellschaft zur Förderung der Wissenschaften e.V., 80539 München (DE)
(72) Erfinder: SKERRA, Arne, D-85354 Freising (DE); WARDENBERG, Christina, D-60323 Frankfurt am Main (DE)
(74) Vertreter: Weiss, Wolfgang, Dipl.-Chem. Dr.
(86) Internationale Anmeldenummer: EP9501862
(87) Internationale Veröffentlichungsnummer: WO95032295

(56) Entgegenhaltungen:
- WO-A-94/04672
- DE-A- 3 934 454
- JOURNAL OF BACTERIOLOGY, Bd. 156, Nr. 3, Dezember 1983 Seite 1188-1191 WRAY, L.V. AND REZNIKOFF, W.S. 'Identification of repressor binding sites controlling expression of tetracycline resistance encoded by Tn10'

### SEQUENZPROTOKOLL

(1) ALLGEMEINE ANGABEN:
   (i) ANMELDER:
      (A) NAME: Max-Planck-Gesellschaft zur Förderung der Wissenschaften e.V.
      (B) STRASSE: Königinstr. 19
      (C) ORT: München
      (E) LAND: Deutschland
      (F) POSTLEITZAHL: 80539
   (ii) BEZEICHNUNG DER ERFINDUNG: Verwendung des Tetracyclinpromotors zur stringent regulierten Produktion von rekombinanten Proteinen in prokaryontischen Zellen
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQÜENZKENNZEICHEN:
      (A) LANGE: 83 Basenpaare
      (B) ART: Nucleotid
      (C) STRANGFORM: beides
      (D) TOPOLOGIE: linear
   (xi) SEQUENZBESCHREIBUNG: SEQ ID NO: 1:
   ATTAATTCCT AATTTTTGTT GACACTCTAT CATTGATAGA GTTATTTTAC CACTCCCTAT 60
   CAGTGATAGA GAAAAGTGAA ATG 83
(2) ANGABEN ZU SEQ ID NO: 2:
   (i) SEQUENZKENNZEICHEN:
      (A) LÄNGE: 3266 Basenpaare
      (B) ART: Nucleotid
      (C) STRANGFORM: Doppelstrang
      (D) TOPOLOGIE: ringförmig
   (xi) SEQUENZBESCHREIBUNG: SEQ ID NO: 2:
   ACCCGACACC ATCGAATGGC CAGATGATTA ATTCCTAATT TTTGTTGACA CTCTATCATT 60
   GATAGAGTTA TTTTACCACT CCCTATCAGT GATAGAGAAA AGTGAAATGA ATAGTTCGAC 120
   AAAAATCTAG ATAACGAGGG CAAAAAATGA AAAAGACAGC TATCGCGATT GCAGTGGCAC 180
   TGGCTGGTTT CGCTACCGTA GCGCAGGCCT GAGACCAGAA TTCGAGCTCG GTACCCGGGG 240
   ATCCCTCGAG GTCGACCTGC AGGCAGCGCT TGGCGTCACC CGCAGTTCGG TGGTTAATAA 300
   GCTTGACCTG TGAAGTGAAA AATGGCGCAC ATTGTGCGAC ATTTTTTTTG TCTGCCGTTT 360
   ACCGCTACTG CGTCACGGAT CTCCACGCGC CCTGTAGCGG CGCATTAAGC GCGGCGGGTG 420
   TGGTGGTTAC GCGCAGCGTG ACCGCTACAC TTGCCAGCGC CCTAGCGCCC GCTCCTTTCG 480
   CTTTCTTCCC TTCCTTTCTC GCCACGTTCG CCGGCTTTCC CCGTCAAGCT CTAAATCGGG 540
   GGCTCCCTTT AGGGTTCCGA TTTAGTGCTT TACGGCACCT CGACCCCAAA AAACTTGATT 600
   AGGGTGATGG TTCACGTAGT GGGCCATCGC CCTGATAGAC GGTTTTTCGC CCTTTGACGT 660
   TGGAGTCCAC GTTCTTTAAT AGTGGACTCT TGTTCCAAAC TGGAACAACA CTCAACCCTA 720
   TCTCGGTCTA TTCTTTTGAT TTATAAGGGA TTTTGCCGAT TTCGGCCTAT TGGTTAAAAA 780
   ATGAGCTGAT TTAACAAAAA TTTAACGCGA ATTTTAACAA AATATTAACG TTTACAATTT 840
   CAGGTGGCAC TTTTCGGGGA AATGTGCGCG GAACCCCTAT TTGTTTATTT TTCTAAATAC 900
   ATTCAAATAT GTATCCGCTC ATGAGACAAT AACCCTGATA AATGCTTCAA TAATATTGAA 960
   AAAGGAAGAG TATGAGTATT CAACATTTCC GTGTCGCCCT TATTCCCTTT TTTGCGGCAT 1020
   TTTGCCTTCC TGTTTTTGCT CACCCAGAAA CGCTGGTGAA AGTAAAAGAT GCTGAAGATC 1080
   AGTTGGGTGC ACGAGTGGGT TACATCGAAC TGGATCTCAA CAGCGGTAAG ATCCTTGAGA 1140
   GTTTTCGCCC CGAAGAACGT TTTCCAATGA TGAGCACTTT TAAAGTTCTG CTATGTGGCG 1200
   CGGTATTATC CCGTATTGAC GCCGGGCAAG AGCAACTCGG TCGCCGCATA CACTATTCTC 1260
   AGAATGACTT GGTTGAGTAC TCACCAGTCA CAGAAAAGCA TCTTACGGAT GGCATGACAG 1320
   TAAGAGAATT ATGCAGTGCT GCCATAACCA TGAGTGATAA CACTGCGGCC AACTTACTTC 1380
   TGACAACGAT CGGAGGACCG AAGGAGCTAA CCGCTTTTTT GCACAACATG GGGGATCATG 1440
   TAACTCGCCT TGATCGTTGG GAACCGGAGC TGAATGAAGC CATACCAAAC GACGAGCGTG 1500
   ACACCACGAT GCCTGTAGCA ATGGCAACAA CGTTGCGCAA ACTATTAACT GGCGAACTAC 1560
   TTACTCTAGC TTCCCGGCAA CAATTGATAG ACTGGATGGA GGCGGATAAA GTTGCAGGAC 1620
   CACTTCTGCG CTCGGCCCTT CCGGCTGGCT GGTTTATTGC TGATAAATCT GGAGCCGGTG 1680
   AGCGTGGCTC TCGCGGTATC ATTGCAGCAC TGGGGCCAGA TGGTAAGCCC TCCCGTATCG 1740
   TAGTTATCTA CACGACGGGG AGTCAGGCAA CTATGGATGA ACGAAATAGA CAGATCGCTG 1800
   AGATAGGTGC CTCACTGATT AAGCATTGGT AGGAATTAAT GATGTCTCGT TTAGATAAAA 1860
   GTAAAGTGAT TAACAGCGCA TTAGAGCTGC TTAATGAGGT CGGAATCGAA GGTTTAACAA 1920
   CCCGTAAACT CGCCCAGAAG CTAGGTGTAG AGCAGCCTAC ATTGTATTGG CATGTAAAAA 1980
   ATAAGCGGGC TTTGCTCGAC GCCTTAGCCA TTGAGATGTT AGATAGGCAC CATACTCACT 2040
   TTTGCCCTTT AGAAGGGGAA AGCTGGCAAG ATTTTTTACG TAATAACGCT AAAAGTTTTA 2100
   GATGTGCTTT ACTAAGTCAT CGCGATGGAG CAAAAGTACA TTTAGGTACA CGGCCTACAG 2160
   AAAAACAGTA TGAAACTCTC GAAAATCAAT TAGCCTTTTT ATGCCAACAA GGTTTTTCAC 2220
   TAGAGAATGC ATTATATGCA CTCAGCGCAG TGGGGCATTT TACTTTAGGT TGCGTATTGG 2280
   AAGATCAAGA GCATCAAGTC GCTAAAGAAG AAAGGGAAAC ACCTACTACT GATAGTATGC 2340
   CGCCATTATT ACGACAAGCT ATCGAATTAT TTGATCACCA AGGTGCAGAG CCAGCCTTCT 2400
   TATTCGGCCT TGAATTGATC ATATGCGGAT TAGAAAAACA ACTTAAATGT GAAAGTGGGT 2460
   CTTAAAAGCA GCATAACCTT TTTCCGTGAT GGTAACTTCA CTAGTTTAAA AGGATCTAGG 2520
   TGAAGATCCT TTTTGATAAT CTCATGACCA AAATCCCTTA ACGTGAGTTT TCGTTCCACT 2580
   GAGCGTCAGA CCCCGTAGAA AAGATCAAAG GATCTTCTTG AGATCCTTTT TTTCTGCGCG 2640
   TAATCTGCTG CTTGCAAACA AAAAAACCAC CGCTACCAGC GGTGGTTTGT TTGCCGGATC 2700
   AAGAGCTACC AACTCTTTTT CCGAAGGTAA CTGGCTTCAG CAGAGCGCAG ATACCAAATA 2760
   CTGTCCTTCT AGTGTAGCCG TAGTTAGGCC ACCACTTCAA GAACTCTGTA GCACCGCCTA 2820
   CATACCTCGC TCTGCTAATC CTGTTACCAG TGGCTGCTGC CAGTGGCGAT AAGTCGTGTC 2880
   TTACCGGGTT GGACTCAAGA CGATAGTTAC CGGATAAGGC GCAGCGGTCG GGCTGAACGG 2940
   GGGGTTCGTG CACACAGCCC AGCTTGGAGC GAACGACCTA CACCGAACTG AGATACCTAC 3000
   AGCGTGAGCT ATGAGAAAGC GCCACGCTTC CCGAAGGGAG AAAGGCGGAC AGGTATCCGG 3060
   TAAGCGGCAG GGTCGGAACA GGAGAGCGCA CGAGGGAGCT TCCAGGGGGA AACGCCTGGT 3120
   ATCTTTATAG TCCTGTCGGG TTTCGCCACC TCTGACTTGA GCGTCGATTT TTGTGATGCT 3180
   CGTCAGGGGG GCGGAGCCTA TGGAAAAACG CCAGCAACGC GGCCTTTTTA CGGTTCCTGG 3240
   CCTTTTGCTG GCCTTTTGCT CACATG 3266

## Beschreibung

Die vorliegende Erfindung betrifft einen prokaryontischen Vektor, der eine regulierbare Expressionskontrollsequenz enthält, die durch den Repressor des Tetracyclin-Resistenzgens reprimierbar ist, eine mit diesem Vektor transformierte prokaryontische Zelle und die Verwendung des Vektors oder der Zelle in einem Verfahren zur gentechnischen Herstellung von Polypeptiden in Prokaryonten.

Zur Produktion heterologer Proteine in E.coli haben sich induzierbare Promotorsysteme als sehr geeignet erwiesen, da eine Synthese des rekombinanten Genprodukts oft eine toxische Wirkung für die Bakterienzelle zeigt, wodurch deren Wachstum und Lebensfähigkeit beeinträchtigt wird. Insbesondere bei Sekretion des Fremdproteins in den periplasmatischen Raum der Wirtszelle ist es empfehlenswert, eine strenge Repression des Promotors nicht nur im Verlauf der Arbeiten zur Herstellung des Vektors sicherzustellen, sondern auch um hohe Zelldichten mit den transformierten Bakterien vor der eigentlichen Proteinproduktion zu erreichen. Die bakterielle Sekretion von Antikörperfragmenten ist ein typisches Beispiel in dieser Hinsicht, da aufgrund der heterologen Genexpression eine toxische und lytische Wirkung auf die Bakterienzelle beobachtet wird (Plückthun und Skerra, Methods Enzymol. 178 (1989), 497-515).

Ein besonders häufig verwendetes induzierbares Promotorsystem ist der durch Isopropyl-β-D-thiogalactopyranosid (IPTG) induzierbare lac-Promotor und seine Derivate, z.B. die Mutante lacUV5 oder der tac-Fusionspromotor (Reznikoff und Gold (1986), in: Maximizing gene expression, Butterworth Publishers, Stoneham, MA). Die Verwendung des lac-Promotors zur gentechnischen Herstellung von Antikörperfragmenten in Bakterien ist beispielsweise bei Skerra (Dissertation, LMU München, Fakultät für Chemie und Pharmazie, 1983) beschrieben.

Die Stärke der Transkription vom lac-Promotor ist jedoch mit dem Genotyp und dem Stoffwechsel der Wirtszelle über die endogene Konzentration von lac-Repressormolekülen einerseits und über die Katabolit-Repressionswirkung andererseits gekoppelt. Man beobachtet daher bei Verwendung eines lac-Expressionssystems erhebliche Variationen der Expressionsstärke mit einem gegebenen Vektor in Abhängigkeit vom verwendeten Wirtsstamm. Dies kann entweder auf eine verringerte Induzierbarkeit zurückzuführen sein - insbesondere, wenn der lac-Repressor sowohl chromosomal als auch Plasmidkodiert ist und daher in zu hohen Mengen vorliegt - oder auf ein Absterben der Zellen bzw. einen Plasmidverlust vor der Induktion aufgrund unzureichender Repression.

Außer dem lac-Promotor sind auch andere regulierbare Promotorsysteme verwendet worden, die meisten davon weisen jedoch erhebliche Nachteile auf, insbesondere wenn eine moderate Sekretion des Fremdgenprodukts oder eine Expression bei verringerter Temperatur zur Begünstigung des Proteinfaltungsprozesses gewünscht wird. Daher besteht ein großes Bedürfnis nach der Bereitstellung einer prokaryontischen Expressionskontrollsequenz, die von individuellen Eigenschaften der bakteriellen Wirtszelle weitgehend entkoppelt ist und die auf einfache und kostengünstige Weise reversibel induzierbar ist.

In einer Arbeit von De la Torre et al. (Plasmid 12 (1984), 103-110) werden Plasmidvektoren beschrieben, in denen die Genexpression durch Tetracyclin partiell reguliert wird. Diese Vektoren enthalten die regulatorische Region des Tetracyclin-Resistenzgens aus dem Transposon Tn10. Diese Region bewirkt ursprünglich die Expression des Tetracyclin-Resistenzgens in einer Richtung und die Expression des Tetracyclin-Repressorstrukturgens in die andere (Bertrand et al., Gene 23 (1983), 149-156). Das Tetracyclin-Repressorprotein unterbindet bei Wechselwirkung mit dem Operator die Expression in beiden Richtungen und unterliegt damit der Autoregulation. Die Repression eines Fremdgens anstelle des Tetracyclin-Resistenzgens unter Kontrolle dieses Tetracyclin-Promotors erfolgte durch Kotransformation mit einem kompatiblen Plasmid, welches das Tetracyclin-Repressorgen ebenfalls unter Kontrolle des Tetracyclin-Promotors enthält. In Wirtszellen, welche diese beiden Plasmide enthalten, konnte bei Abwesenheit von Tetracyclin eine bis zu 8-4fache Repression der Expression eines Fremdgens gefunden werden. Eine derartige geringe Repression ist für die gentechnische Herstellung von Proteinen mit für die Bakterienzelle toxischer Wirkung völlig unzureichend, so daß dieses System bis heute keinerlei praktische Anwendung gefunden hat.

DE 39 34 454 beschreibt regulierbare Expressionsvektoren für Bacillus. Das Tetracyclin-Repressor-Strukturgen befindet sich jedoch stets unter autogener Kontrolle d.h. unter Kontrolle seines eigenen Promotors, der an Bacillus adaptiert wurde, aber immer noch durch das Tetracyclin-Repressor-Protein reprimierbar ist.

Eine der vorliegenden Erfindung zugrundeliegende Aufgabe besteht darin, ein Expressionsvektorsystem zur Herstellung rekombinanter Proteine in prokaryontischen Organismen bereitzustellen, bei dem die Nachteile des Standes der Technik mindestens teilweise beseitigt sind.

Diese Aufgabe wird gelöst durch Bereitstellung eines prokaryontischen Vektors, umfassend (a) eine regulierbare Expressionskontrollsequenz, die durch ein Tetracyclin-Repressorprotein reprimierbar ist und (b) ein Tetracyclin-Repressorstrukturgen in operativer Verknüpfung mit einer Expressionskontrollsequenz, die nicht durch ein Tetracyclin-Repressorprotein reprimierbar ist.

Der erfindungsgemäße Vektor unterscheidet sich von dem aus dem Stand der Technik bekannten Tetracyclin-Expressionssystem dadurch, daß der Tetracyclin-Repressor unter Kontrolle eines unabhängigen Promotors gestellt ist und somit seine eigene Synthese nicht mehr kontrollieren kann. Dadurch wird überraschenderweise eine drastisch verbesserte Repression bei der Expression eines Fremdgens unter Kontrolle der regulierbaren Expressionskontrollsequenz erreicht. Vorzugsweise befindet sich ein Tetracyclin-Repressorstrukturgen, z.B. das Tetracyclin-Repressorgen aus dem Transposon Tn10 (Bertrand et al., Gene 23 (1983), 149-156), auf dem erfindungsgemäßen Vektor in operativer Verknüpfung mit einer konstitutiven Expressionskontrollsequenz, d.h. mit einer nicht-regulierbaren Expressionskontrollsequenz, beispielsweise mit dem Promotor des für die Resistenz gegen Ampicillin verantwortlichen β-Lactamasegens.

Das erfindungsgemäße Tetracyclin-Expressionssystem zeigt im nicht-induzierten Zustand eine erheblich bessere Repression als das lac-Expressionssystem. Dies zeigt sich in einem erheblich besseren Wachstumsverhalten und in einer erhöhten Lebensfähigkeit von Bakterien, die mit einem Gen transformiert sind, das für ein für den Wirtsorganismus toxisches Polypeptid kodiert, wie z.B. ein Antikörperfragment. Ein weiterer Vorteil des erfindungsgemässen Expressionssystems besteht darin, daß die Expression eines heterologen Polypeptids in den unterschiedlichsten E.coli-Wirtsstämmen nahezu identisch ist. Darüber hinaus wurde auch kein signifikanter Einfluß des Nährmediums auf die Expression des heterologen Polypeptids beobachtet. Diese Vorteile des erfindungsgemässen Expressionssystems sind bereits bei Versuchen im Labormaßstab deutlich erkennbar, sie treten jedoch noch in erheblich größerem Umfang bei Versuchen im Fermenter-Maßstab auf. Das erfindungsgemäße Expressionssystem kann daher erfolgreich zur rekombinanten Herstellung von Polypeptiden unter industriellen Bedingungen eingesetzt werden.

Der erfindungsgemäße Vektor ist ein prokaryontischer Vektor, d.h. ein Vektor, der zur Propagierung in einer prokaryontischen Wirtszelle fähig ist. Beispiele für derartige Vektoren sind Plasmidvektoren, Bakteriophage Lambda-Vektoren, Cosmidvektoren und einzelsträngige, filamentöse Bakteriophagenvektoren (vgl. Sambrook et al., Molecular Cloning. A Laboratory Manual, 2nd Ed., Cold Spring Larbor Laboratory Press (1989), Kap. 1-4 und 17). Vorzugsweise ist der erfindungsgemäße Vektor ein zirkuläres Plasmid, insbesondere ein Multi-Copy-Plasmid, das in mehr als 10 Kopien in der Wirtszelle vorliegt. Der Plasmidvektor weist einen für die jeweilige prokaryontische Wirtszelle geeigneten Replikationsursprung auf, z.B. für E.coli einen ColE1- oder p15A-Replikationsursprung. Weiterhin enthält der erfindungsgemäße Vektor vorzugsweise ein Antibiotikumresistenzgen, z.B. ein Ampicillin-, Kanamycin- oder Chloramphenicol-Resistenzgen, um eine Selektion auf Wirtszellen zu ermöglichen, welche den Vektor enthalten.

Der erfindungsgemäße Vektor enthält eine regulierbare Expressionskontrollsequenz, die durch den Repressor des Tetracyclin-Resistenzgens reprimierbar ist. Der Tetracyclin-Repressor ist ein Protein, das in Abwesenheit von Tetracyclin oder Tetracyclinderivaten an den regulatorischen Bereich der Tetracyclin-Promotorregion bindet und die Expression durch diesen Promotor reprimiert. In Anwesenheit von Tetracyclin oder Tetracyclinderivaten wird die Repressorwirkung aufgehoben (Degenkolb et al., Antimicrob. Agents Chemother. 35 (1991), 1591-1595).

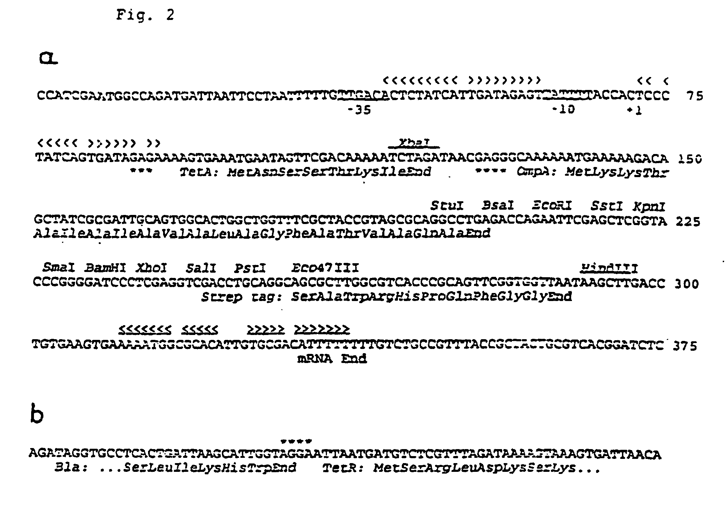

Vorzugsweise enthält die durch ein Tetracyclin-Repressorprotein reprimierbare Expressionskontrollsequenz des erfindungsgemäßen Vektors die in SEQ ID NO. 1 dargestellte Nukleotidsequenz oder eine funktionelle Variante davon. Die in SEQ ID NO. 1 dargestellte Nukleotidsequenz entspricht den Nukleotiden 19 bis 101 in Fig. 2a. Der Begriff "funktionelle Variante" bedeutet, daß die Expressionskontrollsequenz mindestens eine, vorzugsweise zwei funktionelle Tetracyclin-Repressor-Bindestellen enthält, die z.B. zwischen den Positionen -35 und -10 oder +1 bis +19 der Expressionskontrollsequenz (bezüglich der Transkriptionsstartstelle) angeordnet sein können (vgl. Fig. 2a).

Die regulierbare Expressionskontrollsequenz des erfindungsgemäßen Vektors enthält als funktionelle Tetracyclin-Repressor-Bindestellen besonders bevorzugt die palindromen Abschnitte zwischen den Nukleotiden -31 bis -13 oder/und +1 bis +19 (bezüglich der Transkriptionsstartstelle) der in SEQ ID NO. 1 und Fig. 2a gezeigten Nukleotidsequenz mit den Basenfolgen 5'-ACTCTATCATTGATAGAGT-3' und 5'-TCCCTATCAGTGATAG-3' oder DNA-Sequenzen mit äquivalenten Bindungseigenschaften für den Tetracyclin-Repressor.

Weiterhin umfaßt der erfindungsgemäße Vektor vorzugsweise eine multiple Klonierungsstelle in operativer Verknüpfung mit der durch ein Tetracyclin-Repressorprotein reprimierbaren Expressionskontrollsequenz. In diese multiple Klonierungsstelle, die auch als Polylinker bezeichnet wird und vorzugsweise mehrere für den jeweiligen Vektor singuläre Restriktionsschnittstellen enthält, können Fremdgene einkloniert werden, die durch das erfindungsgemäße Expressionssystem exprimiert werden sollen.

Der erfindungsgemäße Vektor kann auch eine Signalpeptid-kodierende Sequenz umfassen, die in operativer Verknüpfung mit der durch ein Tetracyclin-Repressorprotein reprimierbaren Expressionskontrollsequenz ist. Beispiele für geeignete Signalseguenzen sind die OmpA- und die PhoA-Signalsequenzen. Weitere geeignete Signalsequenzen werden beispielsweise von Winnacker in Gene und Klone, Eine Einführung in die Gentechnologie (1985), VCH-Verlagsgesellschaft mbH, Weinheim, auf den Seiten 254 ff. bzw. von Watson "Compilation of published signal sequences" Nucl. Acids Res. 12 (1984), 5145-5164 beschrieben.

Durch Fusionierung der Signalpeptid-kodierenden Sequenz mit einem in den Vektor inserierten Fremdgen wird eine Sekretion des vom Fremdgen kodierten Genprodukts in das Periplasma der Wirtszelle unter Abspaltung des Signalpeptids erreicht.

heiterhin umfaßt der erfindungsgemäße Vektor vorzugsweise eine Transkriptionsterminationssequenz in operativer Verknüpfung mit der durch ein Tetracyclin-Repressorprotein reprimierbaren Expressionkontrollsequenz, wobei die Transkriptionsterminationssequenz, beispielsweise der Lipoprotein-Transkriptionsterminator, auf dem erfindungsgemäßen Vektor derart angeordnet ist, daß die Transkription eines unter Expressionskontrolle des Tetracyclin-Promotors transkribierten Gens am Terminator endet.

Um die Reinigung eines heterologen Polypeptids zu erleichtern, welches durch das Tetracyclin-Expressionssystem erzeugt wird, kann der erfindungsgemäße Vektor weiterhin eine für ein Affinitätspeptid kodierende Nukleotidsequenz in operativer Verknüpfung mit der durch ein Tetracyclin-Repressorprotein reprimierbaren Expressionskontrollsequenz umfassen. Die für das Affinitätspeptid kodierende Sequenz ist im erfindungsgemäßen Vektor zweckmäßigerweise derart lokalisiert, daß sie mit dem C-Terminus eines in den Vektor inserierten Fremdgens fusioniert wird. Beispiele für geeignete Affinitätspeptide sind ein Oligo-Histidin-Peptid, insbesondere eine Sequenz von 5 oder 6 aufeinanderfolgenden Histidin-Resten, das eine Reinigung von bakteriell exprimierten Fremdproteinen durch Metallchelat-Affinitätschromatographie erlaubt (Hochuli et al., Bio/Technology (1988), 1321-1325), oder ein Streptavidin-Bindungspeptid (Schmidt und Skerra, Protein Eng. 6 (1993), 109-122), das eine Reinigung des Fremdproteins durch Affinitätschromatographie mit Streptavidin-Agarose unter Verwendung von sehr milden Elutionsbedingungen gestattet. Ein besonders bevorzugtes, auch als "Strep-tag" bezeichnetes Streptavidin-Bindungspeptid weist die Aminosäuresequenz Ser-Ala-Trp-Arg-His-Pro-Gln-Phe-Gly-Gly auf.

Ein weiterer Gegenstand der vorliegenden Erfindung ist ein Vektor, der mindestens ein für ein heterologes Polypeptid kodierendes Fremdgen in operativer Verknüpfung mit der durch ein Tetracyclin-Repressorprotein reprimierbaren Expressionskontrollsequenz enthält. Gegebenenfalls kann das Fremdgen als Fusion mit einer Signalpeptid-kodierenden Sequenz oder/und einer Affinitätspeptid-kodierenden Sequenz vorliegen. Der Begriff "heterolog" bedeutet, daß das Fremdgen weder für das Tetracyclin-Resistenzprotein noch für das Tetracyclin-Repressorprotein kodiert. Vorzugsweise kodiert das Gen für ein Polypeptid, das für eine prokaryontische Zelle toxisch ist und dessen Expression im nicht-induzierten Zustand möglichst gering gehalten werden muß. Beispiele für derartige toxische Produkte sind Antikörperfragmente. Das erfindungsgemäße Expressionssystem ist jedoch nicht nur für die genannten Beispiele, sondern generell zur Expression beliebiger Proteine, insbesondere Säugerproteine, einsetzbar.

Der erfindungsgemäße Vektor kann auch mehrere Fremdgene unter Kontrolle eines Tetracyclin-Promotors enthalten. Ein Beispiel hierfür ist der Expressionsvektor pASK85-D1.3 (vgl. Fig. 3), der die Gene für die schwere und die leichte Kette eines Antikörper-Fab-Fragments unter Kontrolle des Tetracyclin-Promotors enthält.

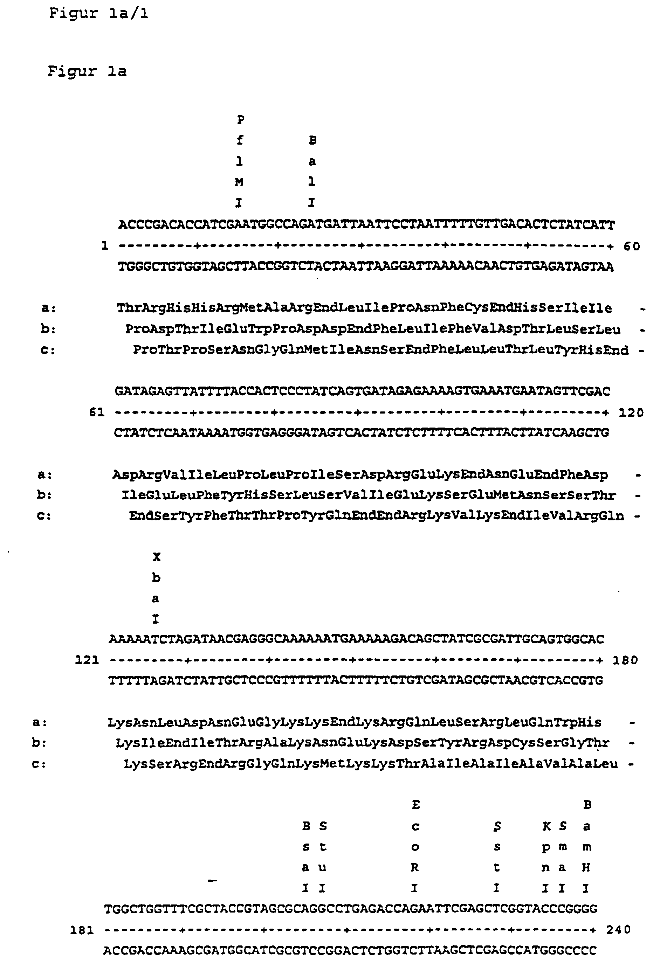

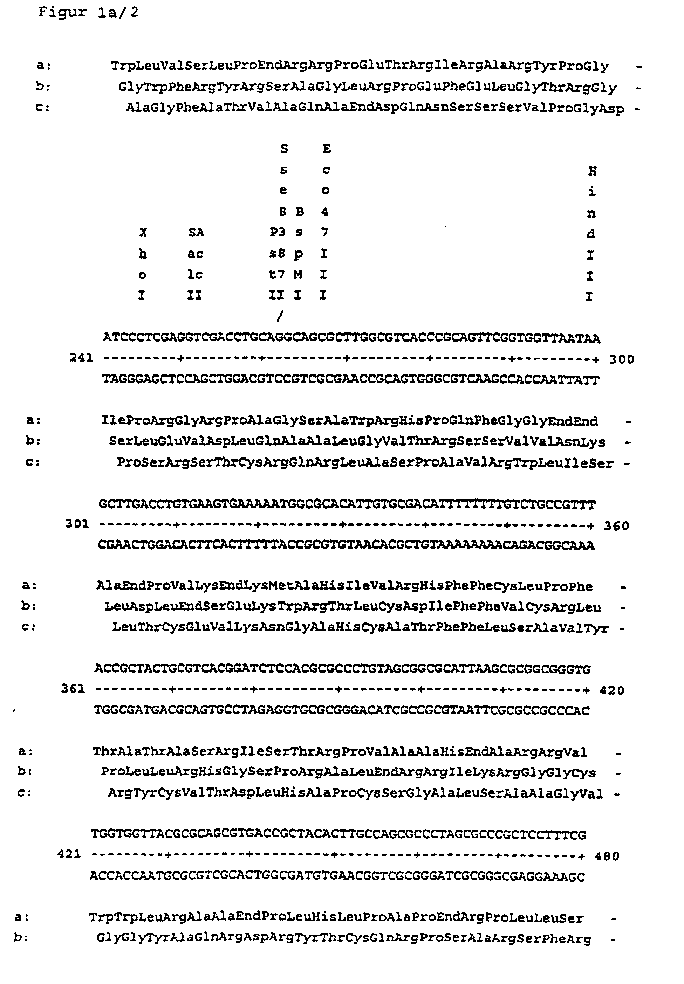

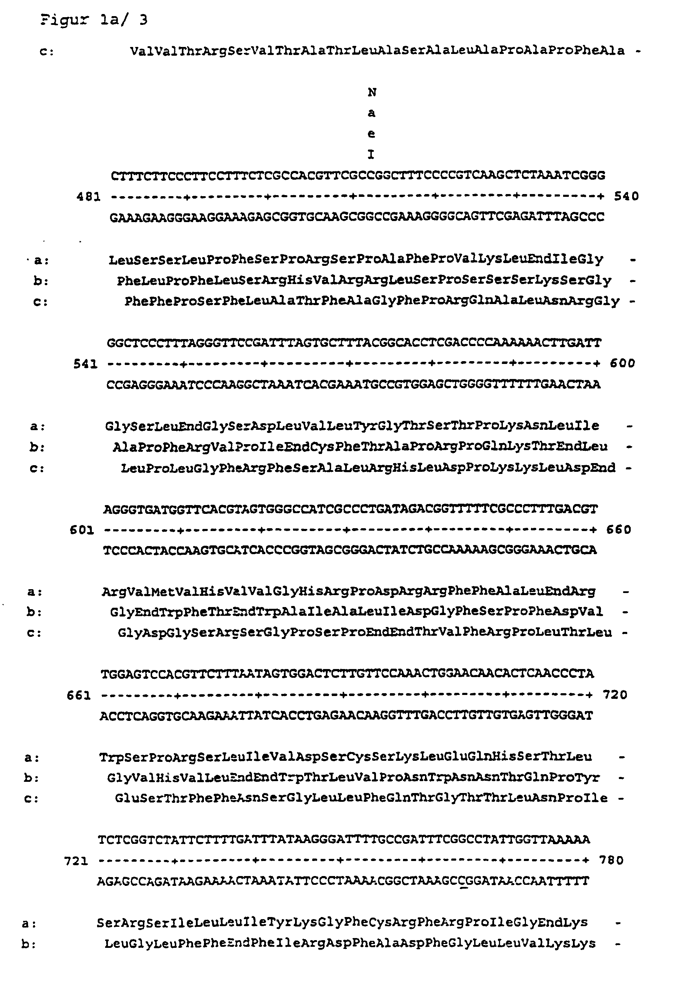

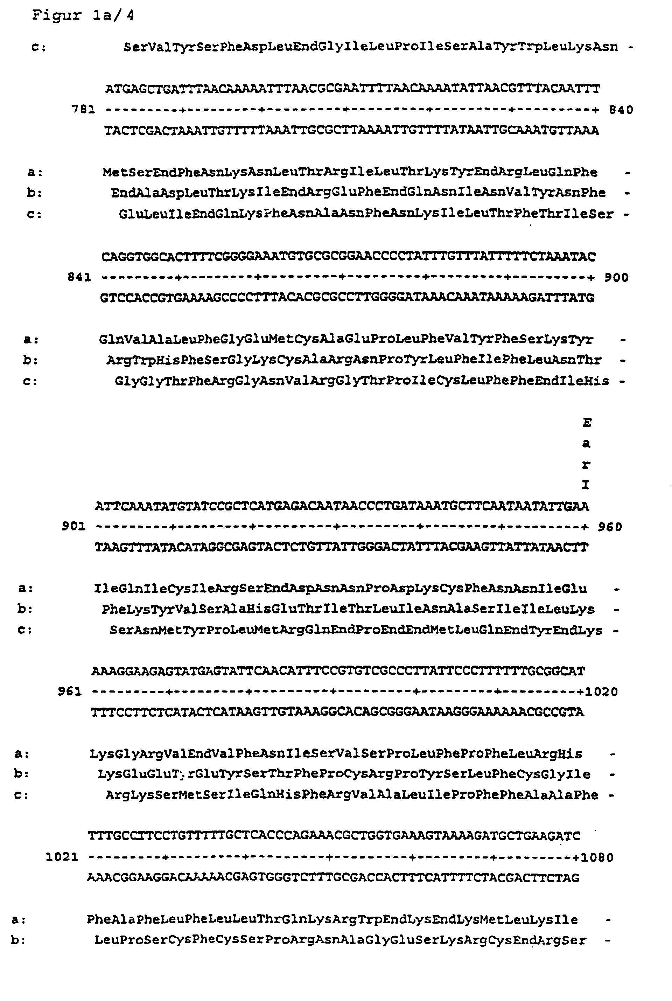

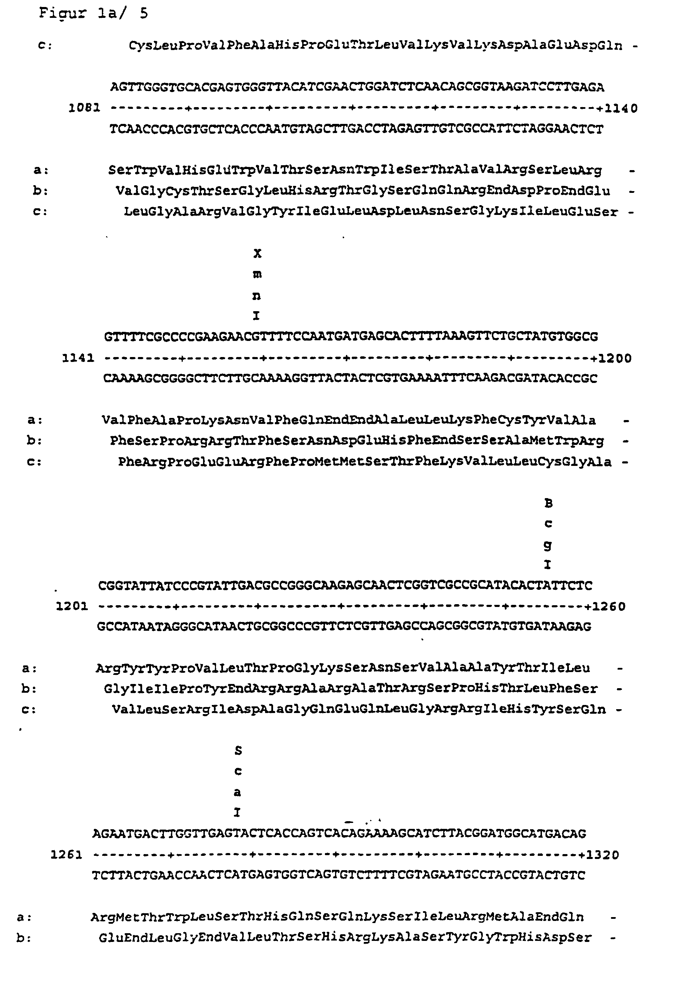

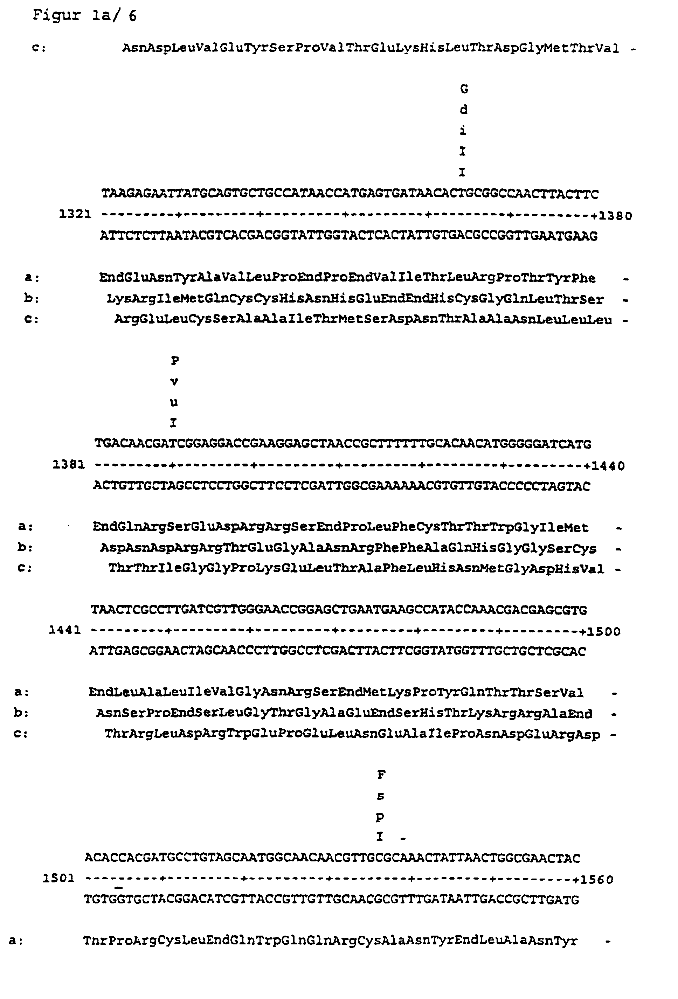

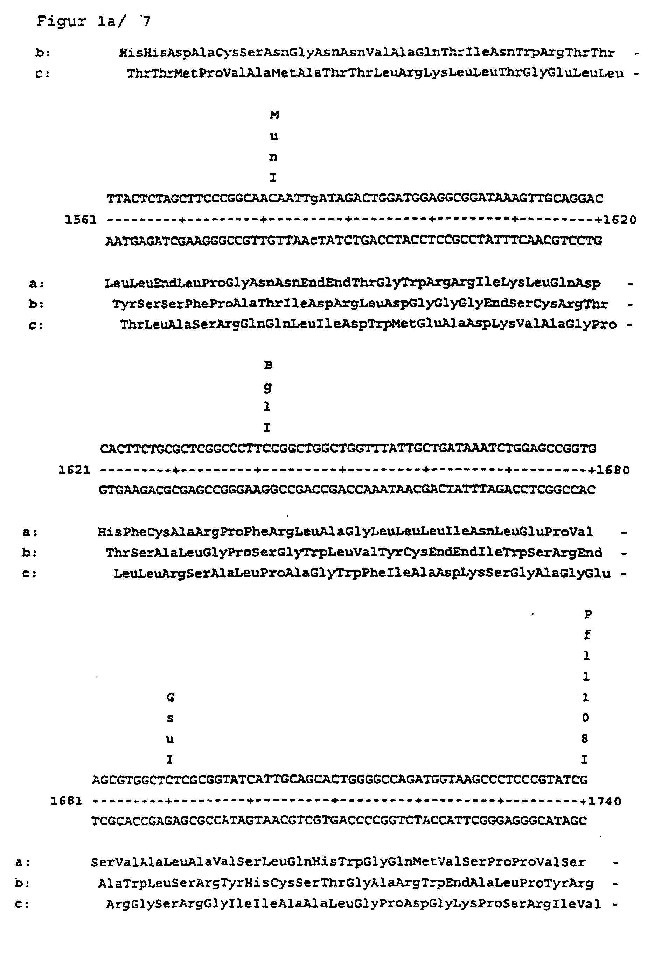

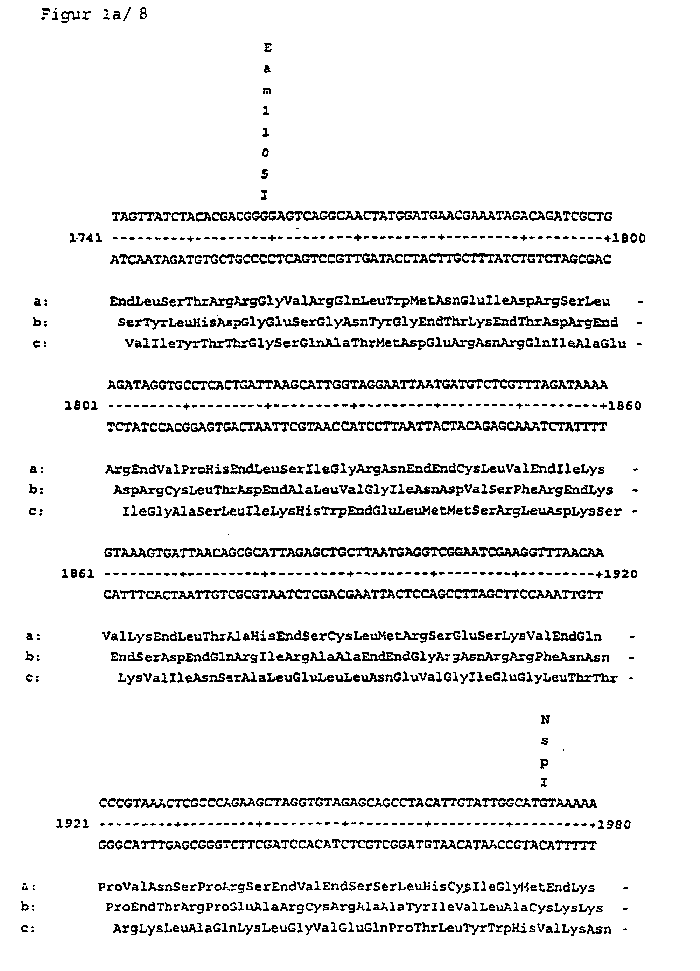

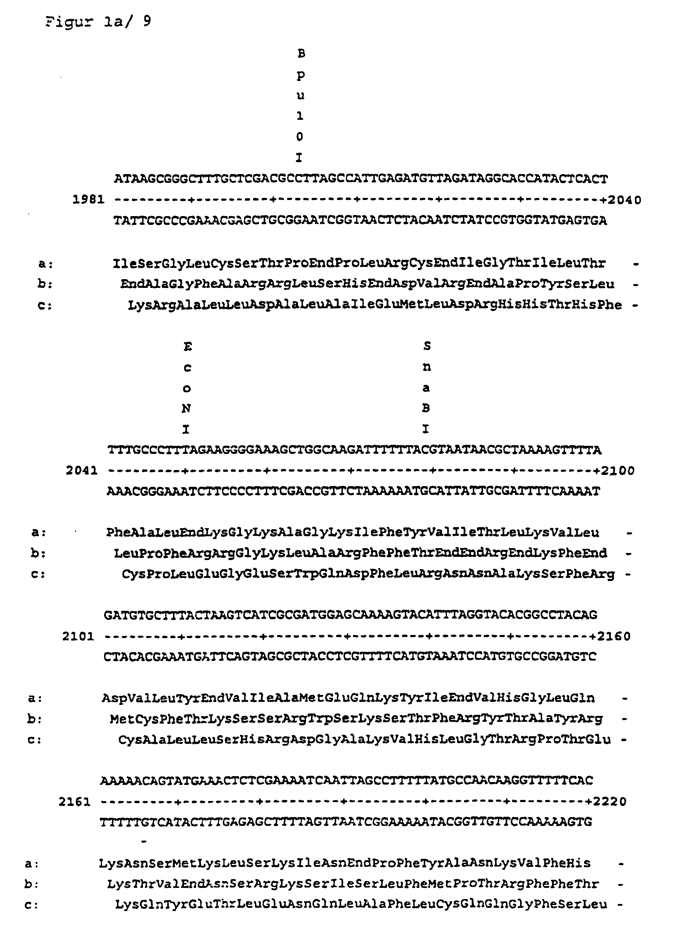

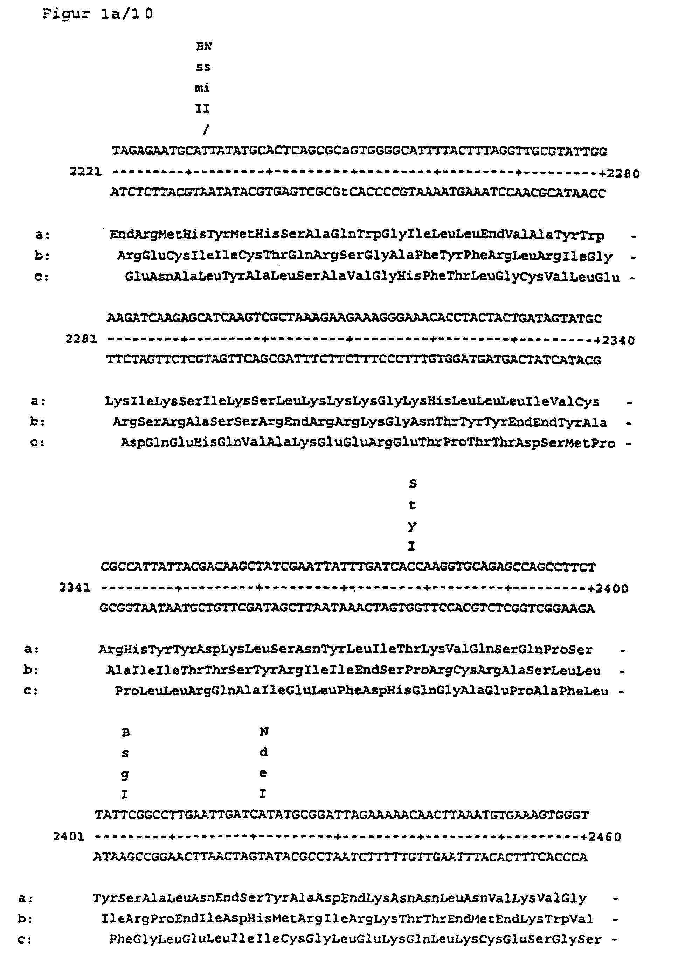

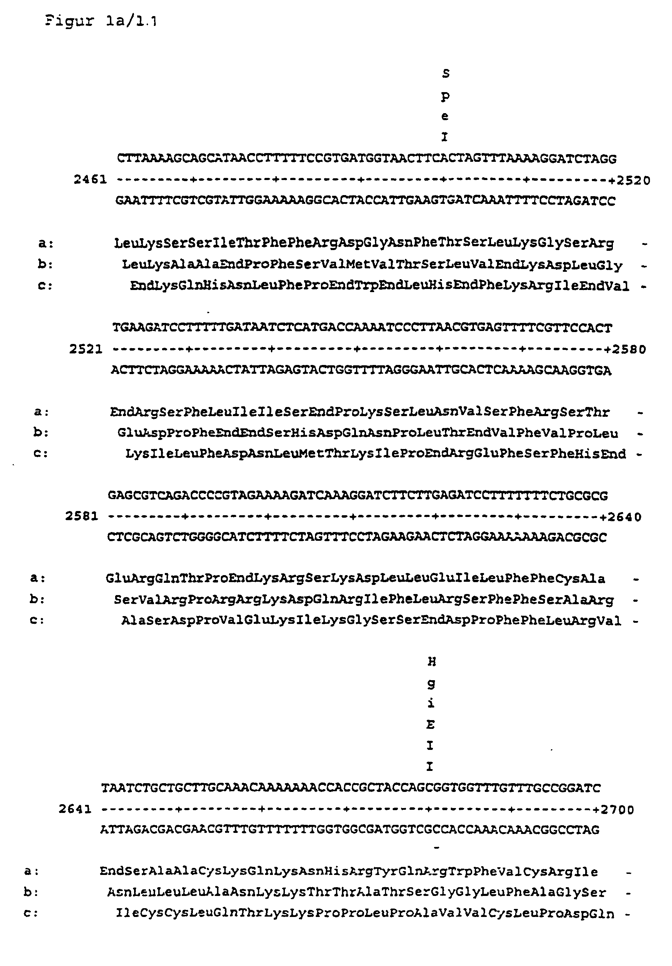

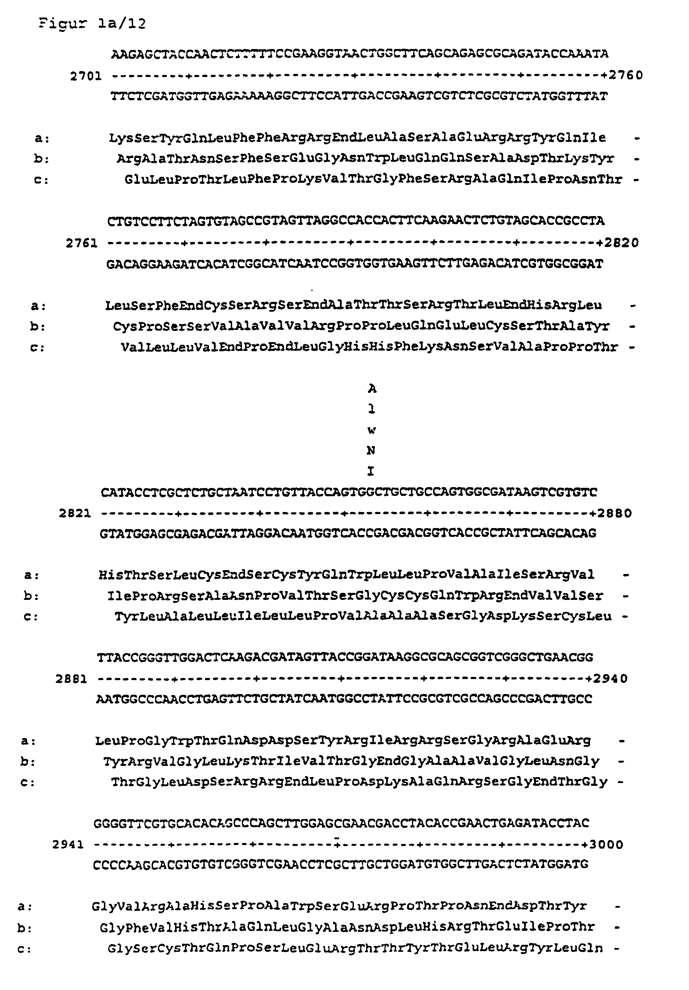

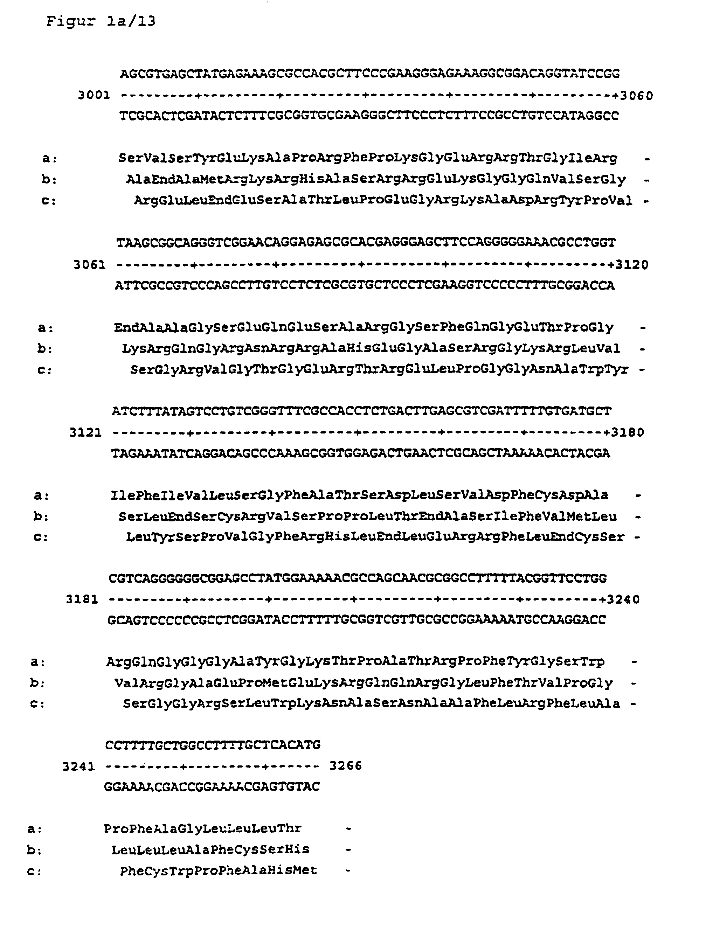

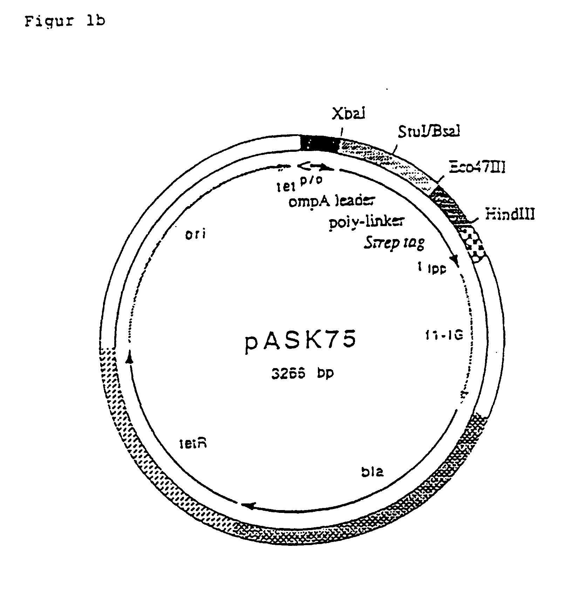

Ein besonders bevorzugtes Beispiel für einen erfindungsgemäßen Vektor ist das Plasmid pASK75, welches die in Fig. 1a und SEQ ID NO. 2 angegebene Nukleotidsequenz aufweist und in Fig. 1b schematisch dargestellt ist. Dieses Plasmid enthält als genetische Elemente einen ColEI-DNA-Replikationsursprung (ori), das β- Lactamasegen (bla), das Strukturgen des Tetracyclin-Repressors (tetR) unter Kontrolle des β-Lactamasepromotors, die intergenische Region des filamentösen Phagen f1 (f1-IG), die Promotor/Operator-Region des Tetracyclin-Resistenzgens aus dem Transposon Tn10 (tetP/O), eine für das OmpA-Signalpeptid kodierende DNA-Sequenz, einen Polylinker, eine für das Streptavidin-Bindungspeptid kodierende DNA-Sequenz (Strep-tag) und den Lipoprotein-Transkriptionsterminator (tₗₚₚ). Diese genetischen Elemente stehen miteinander in operativer Verknüpfung, so daß bei geeigneter Inserierung eines Fremdgens in den Polylinker ein Expressionsvektor bereitgestellt wird, der eine effiziente Expression von Fremdgenen in prokaryontischen Wirtszellen ermöglicht, selbst wenn die vom Fremdgen kodierten Polypeptide ein für die Zelle toxisches Genprodukt darstellen.

Die Erfindung betrifft weiterhin eine prokaryontische Zelle, enthaltend (a) ein für ein heterologes Polypeptid kodierendes Gen in operativer Verknüpfung mit einer regulierbaren Expressionskontrollsequenz, die durch ein Tetracyclin-Repressorprotein reprimierbar ist, und (b) ein Tetracyclin-Repressorgen in operativer Verknüpfung mit einer Expressionskontrollsequenz, die nicht durch ein Tetracyclin-Repressorprotein reprimierbar ist. Vorzugsweise ist diese Zelle mit mindestens einer Kopie eines erfindungsgemäßen Vektors transformiert. Vorzugsweise ist die prokaryontische Zelle eine gram-negative Zelle, besonders bevorzugt eine Enterobakterienzelle (z.B. Salmonella, Escherichia) und am meisten bevorzugt eine E.coli-Zelle.

Der erfindungsgemäße Vektor und die erfindungsgemäße Zelle können in einem Verfahren zur gentechnischen Herstellung von Polypeptiden in Prokaryonten verwendet werden.

Ein weiterer Gegenstand der vorliegenden Erfindung ist ein Verfahren zur gentechnischen Herstellung von Polypeptiden in einer prokaryontischen Zelle, welches dadurch gekennzeichnet ist, daß man
(i) eine Zelle bereitstellt, enthaltend (a) mindestens ein für ein heterologes Polypeptid kodierendes Gen in operativer Verknüpfung mit einer regulierbaren Expressionskontrollsequenz, die durch ein Tetracyclin-Repressorprotein reprimierbar ist, und (b) ein Tetracyclin-Repressorstrukturgen in operativer Verknüpfung mit einer Expressionskontrollsequenz, die nicht durch ein Tetracyclin-Repressorprotein reprimierbar ist,
(ii) die Zelle aus (i) in einem geeigneten Medium unter Bedingungen kultiviert, die zu einer Expression des für das heterologe Polypeptid kodierenden Gens führen, und
(iii) das heterologe Polypeptid aus der Zelle oder dem Medium isoliert.

Das erfindungsgemäße Verfahren kann so durchgeführt werden, daß man eine Wirtszelle verwendet, die die regulierbare Expressionskontrollsequenz und das Tetracyclin-Repressorgen - wie zuvor beschrieben - auf einem einzigen Vektor enthält. Andererseits kann man natürlich auch eine Wirtszelle verwenden, in der sich die regulierbare Expressionskontrollsequenz und das Tetracyclin-Repressorgen nicht auf einem einzigen Vektor befinden, z.B. auf zwei unterschiedlichen, miteinander kompatiblen Vektoren. Noch eine weitere Möglichkeit zur Durchführung des erfindungsgemäßen Verfahrens besteht darin, eine Wirtszelle zu verwenden, die einen Vektor mit dem Fremdgen unter Kontrolle des Tetracyclin-Promotors und eine episomale oder chromosomale Kopie des Tetracyclin-Repressorgens unter Kontrolle eines unabhängigen, nicht durch den Repressor reprimierbaren Promotors enthält.

Die Kultivierung der Zelle in Schritt (ii) des erfindungsgemäßen Verfahrens wird vorzugsweise auf solche Weise durchgeführt, daß bis zum Erreichen einer vorbestimmten Zelldichte die Expression des für das heterologe Polypeptid kodierenden Gens weitgehend reprimiert ist, d.h. in Abwesenheit eines Induktors für die regulierbare Expressionskontrollsequenz, und daß erst nach Erreichen der vorbestimmten Zelldichte die Expression des Fremdgens induziert wird. Bei einer vollständigen Induktion der Expressionskontrollsequenz kann eine maximale Expression von für die Zelle toxischen Genprodukten erreicht werden. Andererseits kann durch Zugabe von geringen Mengen des Induktors eine in manchen Fällen wünschenswerte nur teilweise Induktion der Expressionskontrollsequenz erreicht werden.

Die Induzierung der regulierbaren Expressionskontrollsequenz erfolgt vorzugsweise durch Zugabe von Tetracyclin oder einem Tetracyclin-Derivat als Induktor. Die Zugabe des Induktors bewirkt, daß die Reprimierung der regulierbaren Expressionskontrollsequenz durch das Tetracyclin-Repressorprotein aufgehoben wird.

Als Induktor wird vorzugsweise Anhydrotetracyclin verwendet. Diese Verbindung ist eine kommerziell erhältliche Substanz, die bereits bei extrem geringen Konzentrationen von beispielsweise 5 bis 500 µg Induktor pro Liter Medium wirksam ist und darüber hinaus geringere antibiotische Wirkung zeigt (Oliva et al., Antimicrob. Agents Chemother. 36 (1992), 913-919). Daher ist die Verwendung von Tetracyclin oder Tetracyclin-Derivaten als Induktor auch wirtschaftlicher als die Verwendung von IPTG bei einem Expressionssystem auf Basis des lac-Promotors.

Für eine vollständige Induktion sind Konzentrationen von 100 bis 250 µg/l Anhydrotetracyclin bevorzugt. Für eine teilweise Induktion sind Konzentrationen von 10 bis 50 µg/l Anhydrotetracyclin bevorzugt.

Ein weiterer Vorteil des erfindungsgemäßen Verfahrens besteht darin, daß die gentechnische Herstellung von Polypeptiden mit dem Tetracyclin-Expressionssystem auch in einem Minimalmedium, z.B. einem Glucose-Minimalmedium (vgl. Sambrook et al. (1989), supra, Seite A3) mit hoher Effizienz durchgeführt werden kann. Vorzugsweise werden Minimalmedien verwendet, die neben mineralischen Bestandteilen (z.B. Na⁺, K⁺, Ca²⁺, Mg²⁺, NH₄⁺, Cl⁻, SO₄²⁻, PO₄³⁻) 0,1 bis 10 %, besonders bevorzugt 1 bis 7 % und am meisten bevorzugt 2 bis 5 % einer C-Quelle auf Gewichtsbasis enthalten.

Der Einsatz von Minimalmedien beim erfindungsgemäßen Verfahren kann einerseits derart erfolgen, daß ein NährMedium mit der gesamten gewünschten Menge der C-Quelle vorgelegt wird, in dem die Zellen dann kultiviert werden. Andererseits kann ein Teil der C-Quelle auch später während der Fermentation zudosiert werden, wobei diese Zudosierung vorzugsweise in Abhängigkeit vom Zellwachstum geregelt wird. Eine ausführliche Beschreibung der Kultivierung von transformierten Bakterienzellen in Minimalmedien findet sich bei Riesenberg (Curr. Opin. Biotechnol. 2 (1991), 380-384). Auf die in dieser Literaturstelle beschriebenen Arbeitstechniken wird hiermit Bezug genommen.

Die Erfindung wird weiterhin durch nachfolgende Beispiele, Figuren und Sequenzprotokolle erläutert.

Es zeigen:
- Fig. 1a: die vollständige Nukleotidsequenz des Plasmids pASK75 in doppelsträngiger Form einschließlich der Restriktionsstellen,
- Fig. 1b: eine schematische Darstellung des Vektors pASK75,
- Fig. 2a: die Nukleotidsequenz der gesamten regulatorischen Region auf pASK75,
- Fig. 2b: die künstlich hergestellte intercistronische Region zwischen dem bla-Gen und dem tetR-Strukturgen auf pASK75,
- Fig. 3: eine schematische Darstellung des Vektors pASK85-D1.3,
- Fig. 4a: den Zeitverlauf der Induktion der Synthese eines Antikörper-Fab-Fragments unter Kontrolle eines Tetracyclin-Promotors und
- Fig. 4b: die Abhängigkeit der Synthese des Antikörper-Fab-Fragments vom Wirtsstamm,
- SEQ ID NO. 1: die Nukleotidsequenz des Tetracyclin-Promotor/Operatorbereichs (entsprechend den Nukleotiden 19 bis 101 in Fig. 2a) und
- SEQ ID NO. 2: die vollständige Nukleotidsequenz des Plasmids pASK75.

### BEISPIELE

### Beispiel 1

### Vektorkonstruktion

Der in Fig. 1a und Fig. 1b dargestellte Vektor pASK75, der den Tetracyclin-Promotor/Operatorbereich und das Tetracyclin-Repressorgen enthält, wurde aus dem lac-Promotorplasmid pASK60-Strep (Schmidt und Skerra, Protein. Eng. 6 (1993), 109-122) hergestellt. Die Expressionskassette von pASK75 (die zwischen den XbaI- und HindIII-Restriktionsstellen gelegene DNA-Sequenz, vgl. Fig. 2a) ist identisch wie bei pASK60-Strep und besteht aus einem Genfragment, das für das OmpA-Signalpeptid mit seiner Translationsinitiationsstelle kodiert, einem Polylinker und der für das Streptavidin-Bindungspeptid Strep-tag kodierenden Region.

Die Konstruktion von pASK75 aus pASK60-Strep erfolgte unter Verwendung von Standardmethoden (Sambrook et al. (1989), supra). Zunächst wurde das gesamte Segment auf pASK60-Strep, welches das lacI-Gen und den lac-Promotor/Operator enthielt, durch ein kurzes Fragment aus pWH1012 (Sizemore et al., Nucl. Acids Res. 18 (1990), 2875-2880) mit der Tetracyclin-Promotorregion ersetzt. Das doppelte Methionincodon am 5'-Ende des tetR-Leserahmens wurde zusammen mit der ursprünglich direkt anschließend gelegenen XbaI-Restriktionsstelle entfernt. Das tetR-Strukturgen, das dem Plasmid pWH520 (Berens et al., J. Biol. Chem. 267 (1992), 1945-1952) entnommen wurde, wurde direkt stromabwärts des modifizierten translationalen Stoppkodons für das β-Lactamasegen inseriert. Die XbaI-Restriktionsstelle am Beginn der kodierenden Region wurde durch Mutagenese eliminiert. Stromabwärts des tetR-Leserahmens wurde eine singuläre SpeI-Restriktionsstelle eingeführt und die Eco47III-Stelle im tetR-Strukturgen sowie eine AseI-Stelle im β-Lactamasegen wurden entfernt.

Die Nukleotidsequenz und die Restriktionsstellen in pASK75 sind in Fig. 1a gezeigt. Die Lokalisierung der genetischen Elemente auf pASK75 ist schematisch in Fig. 1b dargestellt. tetP/O ist die Promotor/Operator-Region, Strep-tag ist die für ein Streptavidin-Bindungspeptid kodierende Nukleotidsequenz, tₗₚₚ ist der Lipoprotein-Transkriptionsterminator, bla das β-Lactamasegen, tetR das Strukturgen des tet-Repressors, ori ein ColE1-Replikationsursprung, f1-IG die intergenische Reaktion aus dem filamentösen Phasen f1. Weiterhin sind in Fig. 1b die singulären Restriktionsstellen für XbaI und HindIII, welche die Expressionskassette flankieren, sowie die zur Insertion von Fremdgenen zwischen der OmpA-Signalsequenz und dem Strep-tag-Affinitätspeptid bevorzugt geeigneten Restriktionsstellen angegeben.

pASK75 enthält eine Tandem-Ribosomen-Bindestelle für eine effiziente Translationsinitiation. Ein Strukturgen kann mit der OmpA-Signalsequenz (z.B. über die StuI- oder BsaI-Restriktionsstelle) fusioniert werden, wodurch eine Sekretion des Genprodukts in das Periplasma ermöglicht wird. Andererseits kann das Strukturgen auch in die XbaI-Stelle unter gleichzeitiger Rekonstruktion der zweiten Translationsinitiationsstelle für eine Expression ins Cytoplasma inseriert werden.

Fig. 2a zeigt die Nukleotidsequenz des vollständigen regulatorischen Bereichs auf pASK75, beginnend mit dem tetA-Promotor, bei dem die "-35" und "-10" Konsensussequenzen sowie die Startstelle der Transkription zu erkennen sind. Die den zwei Tetracyclin-Repressor-Bindestellen im Promotorbereich entsprechenden palindromen Muster in der Promotorregion und die Terminatorstruktur sind durch offene Klammern über der Nukleotidsequenz dargestellt. Die Shine-Dalgarno-Elemente sind durch Sternchen markiert.

Das Tetracyclin-Repressorgen ist auf pASK75 vom Tetracyclin-Promotor entkoppelt. Zu diesem Zweck wurde das Strukturgen des Tetracyclin-Repressors einschließlich seiner Shine Dalgarno-Sequenz direkt stromabwärts des konstitutiv exprimierten β-Lactamasegens inseriert, was zu einer transkriptionalen Fusion führt.

Fig. 2b zeigt die künstliche intercistronische Region zwischen dem bla-Gen und dem tetR-Strukturgen auf pASK75. Die Shine-Dalgarno-Sequenz des tetR-Gens ist durch Sternchen gekennzeichnet.

### Beispiel 2

### Genexpression

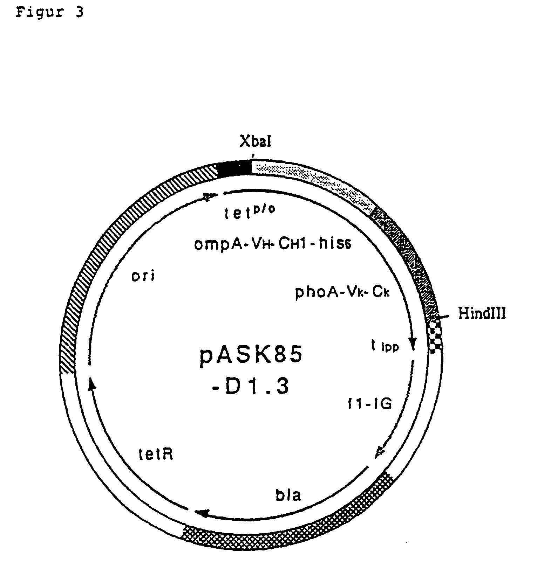

Die Eigenschaften des Tetracyclin-Expressionssystems wurden am Beispiel der Sekretion von Antikörperfragmenten in E.coli untersucht. Als Expressionsvektor wurde das Plasmid pASK85-D1.3 verwendet, das die Strukturgene für die zwei Polypeptidketten eines Fab-Antikörperfragments mit den variablen Domänen des Anti-Lysozym-Antikörpers D1.3 (Boulot et al., J. Mol. Biol. 213 (1990), 617-619) enthält. Sowohl das Gen für die schwere Kette als auch das Gen für die leichte Kette befinden sich jeweils unter Vorschaltung eines bakteriellen Signalpeptids (OmpA, PhoA) unter der gemeinsamen transkriptionalen Kontrolle des Tetracyclin-Promotor/Operators. Das Expressionsplasmid pASK85-D1.3 wurde aus dem Basisvektor pASK75 unter Verwendung des Plasmids pASK84-D1.3 (Skerra, Gene 141 (1994), 79-84) hergestellt. Fig. 3 zeigt eine schematische Darstellung des Plasmids pASK85-D1.3. OmpA-V_{H}-C_{H}1-his6 ist ein Bereich, der für ein Fusionspolypeptid, bestehend aus einem OmpA-Signalpeptid, dem Fragment der schweren Antikörperkette und einer C-terminalen His₆-Sequenz kodiert. PhoA-V_{K}-C_{K} ist ein Bereich, der für ein Fusionspolypeptid, bestehend aus einem PhoA-Signalpeptid und dem Fragment der leichten Antikörperkette, kodiert. Die anderen genetischen Elemente besitzen die gleiche Bedeutung wie in Fig. 1b angegeben.

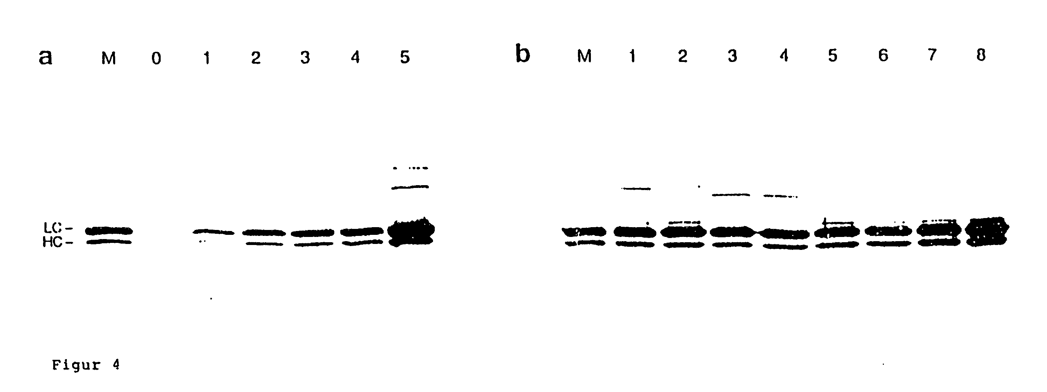

In Fig. 4a ist in einem Western Blot des Gesamtzellproteins der Zeitverlauf einer Induktion der Antikörperfragmentsynthese durch pASK85-D1.3 gezeigt. Die Anfärbung der Blots erfolgte unter Verwendung kommerzieller Antiseren (Kaninchen-Anti-Maus-Ig und Schwein-Anti-Kaninchen-Ig-alkalische Phosphatase-Konjugat, Dako, Hamburg, BRD) und der Farbstoffe Nitroblau-Tetrazolium (Sigma, Deisenhofen, BRD) und 5-Brom-4-chlor-3-indolylphosphat, Toluidinsalz (Boehringer Mannheim GmbH, BRD) nach Standardmethoden (Sambrook et al. (1989), supra). Die Zellen wurden zuvor bei 22°C bis zur mittleren Logphase kultiviert und dann wurde der Promotor durch Zugabe von 200 µg Anhydrotetracyclin pro Liter Medium induziert. Eine Stunde danach konnten beide Ketten des Fab-Fragments deutlich nachgewiesen werden. Ihre Menge stieg im Verlauf der Induktionszeit (4 h) stetig an und nahm sogar während weiterer Inkubation über Nacht noch zu. Ein Vergleich mit dem gereinigten rekombinanten Fab-Fragment zeigte, daß beide Fab-Vorläufer quantitativ prozessiert waren. Eine Abschätzung ergab, daß nach 3 bis 4 h Induktion etwa 20 mg/l Ig-Protein synthetisiert worden waren.

Spur 0 von Fig. 4a zeigt eine unmittelbar vor der Induktion entnommene Probe. Die Spuren 1 bis 4 zeigen Proben von 1 bis 4 h nach der Induktion, Spur 5 zeigt eine Probe nach einer Inkubation über Nacht, Spur M zeigt ca. 1 µg gereinigtes rekombinantes Fab-Fragment. LC und HC bedeuten die jeweils leichte bzw. schwere Kette des Ig-Fragments.

Ein Vergleich der Expression des Ig-Proteins durch das erfindungsgemäße Plasmid pASK85-D1.3 mit dem lac-Promotorplasmid pASK84-D1.3 (Skerra (1994), supra) unter Verwendung von E.coli K12-JM83 (Yanisch-Perron et al., Gene 33 (1985), 103-119) als Wirtsstamm zeigte, daß die bei dem tetA-Promotor erhaltene Ausbeute gleich wie bei dem lacUV5-Promotor war. Auch der Zeitverlauf der Expression und die Gesamtmenge an synthetisiertem Protein waren bei beiden Systemen im wesentlichen gleich. Im Falle des lacUV5-Plasmids konnten jedoch geringe Mengen des Fab-Fragments auch in Abwesenheit des Induktors IPTG nachgewiesen werden.

Aufgrund der toxischen Wirkung bei der Sekretion eines Ig-Fragments in E.coli kann das Ausmaß der Repression in einem Promotorsystem auf qualitative Weise anhand der Lebensfähigkeit und des Wachstumsverhaltens der transformierten Bakterien bestimmt werden. Zu diesem Zweck wurden verschiedene Wirtsstämme mit pASK85-D1.3 transformiert. Im Gegensatz zu dem lacUV5-Promotorplasmid pASK84-D1.3 konnten keine Anzeichen von Toxizität, z.B. das Auftreten von Satellitenkolonien auf Ampicillin-Agarplatten oder die Lyse von Übernachtkulturen, bei Verwendung zahlreicher verschiedener E.coli-Stämme gefunden werden. Weiterhin waren die Zelldichten von Übernachtkulturen (bei 37°C) unter Verwendung des Tetracyclin-Promotorplasmids reproduzierbar höher, und die Plasmidpräparationen ergaben durchgehend gute Ausbeuten. Auch die Herstellung großer Mengen von einzelsträngiger Phagemid-DNA war aus den tet-Promotorvektoren mit Standardmethoden möglich, was ein weiteres Anzeichen für die effiziente Repression des Fremdgens darstellt.

Fig. 4b zeigt das Ergebnis von Experimenten zur Expression des rekombinanten Fab-Fragments in verschiedenen E.coli-Wirtsstämmen der Klassen K12 oder B.

Der Nachweis von rekombinantem Fab-Fragment in Fig. 4b erfolgte mittels eines Western Blots des gesamten E.coli-Zellproteins 3 h nach der Induktion wie in Fig. 4a. Spur 1: JM83 (Yanisch-Perron et al. (1985) supra), Spur 2: WK6 (Zell und Fritz, EMBO J. 6 (1987), 1809-1815), Spur 3: E.coli B (ATCC 11303), Spur 4: BL21 (Studier und Moffat (1986), J. Mol. Biol. 189 (1986), 113-130), Spur 5: MG1655 (Jensen, J. Bacteriol. 175 (1993), 3401-3407); Spur 6: W3110 (Jensen (1993), supra); Spur 7: W3110 in Glucose-Minimalmedium, Spur 8: XL1-Blue (Bullock et al., Biotechniques 5 (1987), 376-379); Spur M: ca. 1 µg gereinigtes rekombinantes Fab-Fragment.

Fig. 4b zeigt, daß nahezu identische Mengen des rekombinanten Fab-Fragments unabhängig vom E.coli-Wirtsstamm synthetisiert wurden. Eine volle Induktion des Promotors wurde in diesem System mit 100 oder 200 µg/l Anhydrotetracyclin gefunden. Unter diesen Bedingungen wurden beide Ketten des rekombinanten Fab-Fragments quantitativ prozessiert und auch in das Periplasma sekretiert. Weiterhin ist festzustellen, daß die Expression des rekombinanten Fab-Fragments weder durch Vorhandensein einer episomalen Kopie des Tn10-Tetracyclin-Resistenzgens (Stamm E.coli XL1-Blue) oder bei Kultivierung in einem Glucose-Minimalmedium beeinträchtigt wurde.

### Beispiel 3

### Genexpression in einem 4 l-Fermenter

Die Verwendung von Minimalmedien mit definierter Zusammensetzung stellt besonders günstige Voraussetzungen für die Kultivierung der transformierten Bakterienzellen bis zu hoher Zelldichte im Fermenter dar (Riesenberg, Curr. Opin. Biotechnol. 2 (1991), 380-384). Zu diesem Zweck ist es wünschenswert, mit einem Bakterienstamm zu arbeiten, der keine Auxotrophien aufweist. Die Eigenschaft des erfindungsgemäßen Promotorsystems, weitgehend unabhängig vom Nährmedium sowie den Charakteristika des E.coli-Stamms zu funktionieren, ist in diesem Zusammenhang besonders vorteilhaft.

Die Produktion des künstlichen Antikörper-Fab-Fragments M41 wurde in einem 4-Liter-Fermenter in Gegenwart eines Glucose-Minimalmediums (Sambrook et al., supra), dem Mineralsalze und 100 mg pro Liter Ampicillin zur Selektion auf das Expressionsplasmid zugesetzt waren, untersucht. Das verwendete Expressionsplasmid pASK85-M41 war analog zum Plasmid pASK85-D1.3 aufgebaut, wobei die variablen Domänen des kodierten Antikörper-Fab-Fragments eine andere Sequenz aufwiesen. Durch Plattieren von Proben auf Agar-Kulturplatten in Gegenwart und Abwesenheit von Ampicillin wurde nachgewiesen, daß auch bei hoher Zelldichte kein Verlust des Expressionsplasmids in der Kultur auftrat. Wenn die Genexpression bei einer Zelldichte OD(550) = 10 durch Zugabe von 0,5 mg pro Liter Anhydrotetracyclin 3 h induziert wurde, wurden, bezogen auf die Zellmasse, vergleichbare Ausbeuten des rekombinanten Proteins erhalten wie bei Kultivierung im Schüttelkolben in Gegenwart eines Vollmediums.

## Patentansprüche

1. Prokaryontischer Vektor, umfassend (a) eine regulierbare Expressionskontrollsequenz, die durch ein Tetracyclin-Repressorprotein reprimierbar ist und (b) ein Tetracyclin-Repressor-Strukturgen in operativer Verknüpfung mit einer Expressionskontrollsequenz, die nicht durch ein Tetracyclin-Repressorprotein reprimierbar ist.

2. Vektor nach Anspruch 1,
**dadurch gekennzeichnet,**
**daß** die durch ein Tetracyclin-Repressorprotein reprimierbare Expressionskontrollsequenz die in SEQ ID NO. 1 dargestellte Nukleotidsequenz oder eine funktionelle Variante davon enthält.

3. Vektor Anspruch 1 oder 2,
**dadurch gekennzeichnet,**
**daß** die durch ein Tetracyclin-Repressorprotein reprimierbare Expressionskontrollsequenz die Basenfolgen 5'-ACTCTATCATTGATAGAGT-3' oder/und 5'-TCCCTATCAGTGATAGAGA-3' enthält.

4. Vektor nach einem der Ansprüche 1 bis 3,
**dadurch gekennzeichnet,**
**daß** das Tetracyclin-Repressorgen in operativer Verknüpfung mit einer konstitutiven Expressionskontrollsequenz ist.

5. Vektor nach einem der Ansprüche 1 bis 4,
**dadurch gekennzeichnet,**
**daß** er ein zirkuläres Plasmid ist.

6. Vektor nach einem der Ansprüche 1 bis 5, weiterhin umfassend eine multiple Klonierungsstelle in operativer Verknüpfung mit der durch ein Tetracyclin-Repressorprotein reprimierbaren Expressionskontrollsequenz.

7. Vektor nach einem der Ansprüche 1 bis 6, weiterhin umfassend eine Signalpeptid-kodierende Sequenz in operativer Verknüpfung mit der durch ein Tetracyclin-Repressorprotein reprimierbaren Expressionskontrollsequenz.

8. Vektor nach Anspruch 7,
**dadurch gekennzeichnet,**
**daß** die Signalpeptid-kodierende Sequenz eine OmpA- oder PhoA-Signalsequenz ist.

9. Vektor nach einem der Ansprüche 1 bis 8, weiterhin umfassend eine Transkriptionsterminationssequenz in operativer Verknüpfung mit der durch ein Tetracyclin-Repressorprotein reprimierbaren Expressionskontrollsequenz.

10. Vektor nach Anspruch 9,
**dadurch gekennzeichnet,**
**daß** die Transkriptionsterminationssequenz der Lipoprotein-Transkriptionsterminator ist.

11. Vektor nach einem der Ansprüche 1 bis 10, weiterhin umfassend eine Affinitätspeptid-kodierende Nukleotidsequenz in operativer Verknüpfung mit der durch ein Tetracyclin-Repressorprotein reprimierbaren Expressionskontrollsequenz.

12. Vektor nach Anspruch 11,
**dadurch gekennzeichnet,**
**daß** die Affinitätspeptid-kodierende Nukleotidsequenz für ein Oligo-Histidin-Peptid oder für ein Streptavidin-Bindungspeptid kodiert.

13. Vektor nach einem der Ansprüche 1 bis 12, weiterhin umfassend mindestens ein für ein heterologes Polypeptid kodierendes Strukturgen in operativer Verknüpfung mit der durch ein Tetracyclin-Repressorprotein reprimierbaren Expressionskontrollsequenz.

14. Vektor nach Anspruch 13,
**dadurch gekennzeichnet,**
**daß** *das* Strukturgen für ein Polypeptid kodiert, das für eine prokaryontische Zelle toxisch ist.

15. Vektor nach Anspruch 13 oder 14,
**dadurch gekennzeichnet,**
**daß** das Gen für eine Antikörperfragment-Kette oder das Serum-Retinol-Bindungsprotein kodiert.

16. Plasmid pASK 75 mit der in SEQ ID NO. 2 dargestellten Nukleotidsequenz.

17. Prokaryontische Zelle, enthaltend (a) ein für ein heterologes Polypeptid kodierendes Gen in operativer Verknüpfung mit einer regulierbaren Expressionskontrollsequenz, die durch ein Tetracyclin-Repressorprotein reprimierbar ist, und (b) ein Tetracyclin-Repressorstrukturgen in operativer Verknüpfung mit einer Expressionskontrollsequenz, die nicht durch ein Tetracyclin-Repressorprotein reprimierbar ist.

18. Prokaryontische Zelle nach Anspruch 17,
**dadurch gekennzeichnet,**
**daß** sie mit mindestens einer Kopie eines Vektors nach einem der Ansprüche 1 bis 16 transformiert ist.

19. Zelle nach Anspruch 18,
**dadurch gekennzeichnet,**
**daß** sie eine E.coli-Zelle ist.

20. Verwendung eines Vektors nach einem der Ansprüche 1 bis 16 oder einer Zelle nach einem der Ansprüche 17 bis 19 in einem Verfahren zur gentechnischen Herstellung von Polypeptiden in Prokaryonten.

21. Verfahren zur gentechnischen Herstellung von Polypeptiden in einer prokaryontischen Zelle,
**dadurch gekennzeichnet,**
**daß** man
(i) eine Zelle bereitstellt, enthaltend (a) mindestens ein für ein heterologes Polypeptid kodierendes Gen in operativer Verknüpfung mit einer regulierbaren Expressionskontrollsequenz, die durch ein Tetracyclin-Repressorprotein reprimierbar ist, und (b) ein Tetracyclin-Repressorstrukturgen in operativer Verknüpfung mit einer Expressionskontrollsequenz, die nicht durch ein Tetracyclin-Repressorprotein reprimierbar ist,
(ii) die Zelle aus (i) in einem geeigneten Medium unter Bedingungen kultiviert, die zu einer Expression des für das heterologe Polypeptid kodierenden Gens führen, und
(iii) das heterologe Polypeptid aus der Zelle oder dem Medium isoliert.

22. Verfahren nach Anspruch 21,
**dadurch gekennzeichnet,**
**daß** man eine Zelle verwendet, die die regulierbare Expressionskontrollsequenz und das Tetracyclin-Repressorstrukturgen auf einem einzigen Vektor enthält.

23. Verfahren nach Anspruch 21,
**dadurch gekennzeichnet,**
**daß** man eine Zelle verwendet, die die regulierbare Expressionskontrollsequenz und das Tetracyclin-Repressorstrukturgen auf zwei unterschiedlichen, miteinander kompatiblen Vektoren enthält.

24. Verfahren nach einem der Ansprüche 21 bis 23,
**dadurch gekennzeichnet,**
**daß** die Kultivierung der Zelle in Schritt (ii) zunächst bis zum Erreichen einer vorbestimmten Zelldichte unter derartigen Bedingungen erfolgt, daß die Expression des für das heterologe Polypeptid kodierenden Gens weitgehend reprimiert ist, und daß nach Erreichen der vorbestimmten Zelldichte die Expression des Gens vollständig oder teilweise induziert wird.

25. Verfahren nach Anspruch 24,
**dadurch gekennzeichnet,**
**daß** die Induzierung durch Zugabe von Tetracyclin oder einem Tetracyclinderivat erfolgt.

26. Verfahren nach Anspruch 25,
**dadurch gekennzeichnet,**
**daß** die Induzierung durch Zugabe von Anhydrotetracyclin erfolgt.

27. Verfahren nach Anspruch 24 oder 25,
**dadurch gekennzeichnet,**
**daß** die Zugabe des Induktors bis auf eine Endkonzentration von 5 bis 500 µg/l Medium erfolgt.

28. Verfahren nach einem der Ansprüche 21 bis 27,
**dadurch gekennzeichnet,**
**daß** man die Zelle in einem Minimalmedium kultiviert.

## Claims

1. Prokaryotic vector comprising (a) a regulatable expression sequence which can be repressed by a tetracycline repressor protein and (b) a tetracycline repressor structural gene in operative linkage with an expression control sequence which cannot be repressed by a tetracycline repressor protein.

2. Vector as claimed in claim 1,
**wherein**
the expression control sequence that can be repressed by a tetracycline repressor protein contains the nucleotide sequence shown in SEQ ID NO. 1 or a functional variant thereof.

3. Vector as claimed in claim 1 or 2,
**wherein**
the expression control sequences that can be repressed by a tetracycline repressor protein contains the base sequences 5'-ACTCTATCATTGATAGAGT-3' or/and 5'-TCCCTATCAGTGATAGAGA-3'.

4. Vector as claimed in one of the claims 1 to 3,
**wherein**
the tetracycline repressor gene is in operative linkage with a constitutive expression control sequence.

5. Vector as claimed in one of the claims 1 to 4,
**wherein**
it is a circular plasmid.

6. Vector as claimed in one of the claims 1 to 5 which additionally comprises a multiple cloning site in operative linkage with the expression control sequence that can be repressed by a tetracycline repressor protein.

7. Vector as claimed in one of the claims 1 to 6 which additionally comprises a signal peptide coding sequence in operative linkage with the expression control sequence that can be repressed by a tetracycline repressor protein.

8. Vector as claimed in claim 7,
**wherein**
the sequence coding for the signal peptide is an Ompa or PhoA signal sequence.

9. Vector as claimed in one of the claims 1 to 8 which additionally comprises a transcription termination sequence in operative linkage with the expression control sequence that can be repressed by a tetracycline repressor protein.

10. Vector as claimed in claim 9,
**wherein**
the transcription termination sequence is the lipoprotein transcription terminator.

11. Vector as claimed in one of the claims 1 to 10 which additionally comprises a nucleotide sequence coding for an affinity peptide in operative linkage with the expression control sequence that can be repressed by a tetracycline repressor protein.

12. Vector as claimed in claim 11,
**wherein**
the nucleotide sequence coding for the affinity peptide codes for an oligo-histidine peptide or for a streptavidin binding peptide.

13. Vector as claimed in one of the claims 1 to 12 which additionally comprises at least one structural gene coding for a heterologous polypeptide in operative linkage with the expression control sequence that can be repressed by a tetracycline repressor protein.

14. Vector as claimed in claim 13,
**wherein**
the structural gene codes for a polypeptide which is toxic to a prokaryotic cell.

15. Vector as claimed in claim 13 or 14,
**wherein**
the gene codes for an antibody fragment chain or the serum retinol binding protein.

16. Plasmid pASK75 having the nucleotide sequence shown in SEQ ID NO. 2.

17. Prokaryotic cell containing (a) a gene coding for a heterologous polypeptide in operative linkage with a regulatable expression control sequence which can be repressed by a tetracycline repressor protein and (b) a tetracycline repressor structural gene in operative linkage with an expression control sequence which cannot be repressed by a tetracycline repressor protein.

18. Prokaryotic cell as claimed in claim 17,
**wherein**
it is transformed with at least one copy of a vector as claimed in one of the claims 1 to 16.

19. Cell as claimed in claim 18,
**wherein**
it is an E. coli cell.

20. Use of a vector as claimed in one of the claims 1 to 16 or a cell as claimed in one of the claims 17 to 19 in a process for the production of polypeptides in prokaryotes by genetic engineering.

21. Process for the production of polypeptides in a prokaryotic cell by genetic engineering,
**wherein**
(i) a cell is provided containing (a) at least one gene coding for a heterologous polypeptide in operative linkage with a regulatable expression control sequence that can be repressed by a tetracycline repressor protein and (b) a tetracycline repressor structural gene in operative linkage with an expression control sequence that cannot be repressed by a tetracycline repressor protein,
(ii) the cell from (i) is cultured in a suitable medium under conditions which lead to an expression of the gene coding for the heterologous peptide and
(iii) the heterologous polypeptide is isolated from the cell or form the medium.

22. Process as claimed in claim 21,
**wherein**
a cell is used which contains the regulatable expression control sequence and the tetracycline repressor structural gene on a single vector.

23. Process as claimed in claim 21,
**wherein**
a cell is used which contains a regulatable expression control sequence and the tetracycline repressor structural gene on two different vectors that are compatible with one another.

24. Process as claimed in one of the claims 21 to 23,
**wherein**
in step (ii) the cell is firstly cultured until a predetermined cell density is reached under such conditions that the expression of the gene coding for the heterologous polypeptide is extensively repressed and the expression of the gene is completely or partially induced when the predetermined cell density is reached.

25. Process as claimed in claim 24,
**wherein**
the induction is achieved by addition of tetracycline or a tetracycline derivative.

26. Process as claimed in claim 25,
**wherein**
the induction is achieved by addition of anhydrotetracycline.

27. Process as claimed in one of the claims 24 or 25,
**wherein**
the inducer is added to the medium up to a final concentration of 5 to 500 µg/l medium.

28. Process as claimed in one of the claims 21 to 27,
**wherein**
the cell is cultured in a minimal medium.

## Revendications

1. Vecteur procaryote, comprenant (a) une séquence régulatrice du contrôle de l'expression, qui est réprimable par une protéine répresseur de la tétracycline, et (b) un gène de structure du répresseur de la tétracycline en liaison fonctionnelle avec une séquence du contrôle de l'expression, qui n'est pas réprimable par une protéine répresseur de la tétracycline.

2. Vecteur selon la revendication 1, **caractérisé en ce que** la séquence du contrôle de l'expression, réprimable par une protéine répresseur de la tétracycline, contient la séquence des nucléotides représentée dans SEQ ID NO.1 ou une variante fonctionnelle de celle-ci.

3. Vecteur selon la revendication 1 ou 2, **caractérisé en ce que** la séquence du contrôle de l'expression, réprimable par une protéine répresseur de la tétracycline, contient la suite de bases 5'-ACTCTATCATTGATAGAGT-3' et/ou 5'-TCCCTATCAGTGATAGAGA-3'.

4. Vecteur selon l'une quelconque des revendications 1 à 3, **caractérisé en ce que** le gène répresseur de la tétracycline est en liaison fonctionnelle avec une séquence constitutive du contrôle de l'expression.

5. Vecteur selon l'une quelconque des revendications 1 à 4, **caractérisé en ce qu'**il est un plasmide circulaire.

6. Vecteur selon l'une quelconque des revendications 1 à 5, comprenant en outre, un site multiple de clonage en liaison fonctionnelle avec la séquence du contrôle de l'expression, réprimable par une protéine répresseur de la tétracycline.

7. Vecteur selon l'une quelconque des revendications 1 à 6, comprenant en outre, une séquence codant pour un peptide signal, en liaison fonctionnelle avec la séquence du contrôle de l'expression, réprimable par une protéine répresseur de la tétracycline.

8. Vecteur selon la revendication 7, **caractérisé en ce que** la séquence codant pour un peptide signal est une séquence OmpA ou PhoA.

9. Vecteur selon l'une quelconque des revendications 1 à 8, comprenant en outre, une séquence de terminaison de la transcription en liaison fonctionnelle avec la séquence du contrôle de l'expression, réprimable par une protéine répresseur de la tétracycline.

10. Vecteur selon la revendication 9, **caractérisé en ce que** la séquence de terminaison de la transcription est le terminateur de transcription des lipoprotéines.

11. Vecteur selon l'une quelconque des revendications 1 à 10, comprenant en outre, une séquence de nucléotides, codant pour un peptide d'affinité, en liaison fonctionnelle avec la séquence du contrôle de l'expression, réprimable par une protéine répresseur de la tétracycline.

12. Vecteur selon la revendication 11, **caractérisé en ce que** la séquence de nucléotides codant pour un peptide d'affinité code pour un peptide oligo-histidine ou pour un peptide de liaison de la streptavidine.

13. Vecteur selon l'une quelconque des revendications 1 à 12, comprenant en outre, au moins un gène de structure codant pour un polypeptide hétérologue en liaison fonctionnelle avec la séquence du contrôle de l'expression, réprimable par une protéine répresseur de la tétracycline.

14. Vecteur selon la revendication 13, **caractérisé en ce que** le gène de structure code pour un polypeptide qui est toxique pour une cellule procaryote.

15. Vecteur selon la revendication 13 ou 14, **caractérisé en ce que** le gène code pour une chaîne fragment d'anticorps ou pour la protéine de liaison sérum-rétinol.

16. Plasmide pASK 75, avec la séquence de nucléotides représentée dans SEQ ID NO.2.

17. Cellule procaryote, contenant (a) un gène codant pour un polypeptide hétérologue, en liaison fonctionnelle avec une séquence régulatrice du contrôle de l'expression, qui est réprimable par une protéine répresseur de la tétracycline, et (b) un gène de structure du répresseur de la tétracycline en liaison fonctionnelle avec une séquence du contrôle de l'expression, qui n'est pas réprimable par une protéine répresseur de la tétracycline.

18. Cellule procaryote selon la revendication 17, **caractérisée en ce qu'**elle est transformée avec au moins une copie du vecteur selon l'une quelconque des revendications 1 à 16.

19. Cellule selon la revendication 18, **caractérisé en ce qu'**elle est une cellule *E. coli*.

20. Utilisation d'un vecteur selon l'une quelconque des revendications 1 à 16 ou d'une cellule selon l'une quelconque des revendications 17 à 19, dans un procédé pour la préparation par génie génétique de polypeptides dans des procaryotes.

21. Procédé pour la préparation par génie génétique de polypeptides dans une cellule procaryote, **caractérisé en ce que**
(i) on prépare une cellule, contenant (a) au moins un gène codant pour un polypeptide hétérologue, en liaison fonctionnelle avec une séquence régulatrice du contrôle de l'expression, qui est réprimable par une protéine répresseur de la tétracycline, et (b) un gène de structure du répresseur de la tétracycline en liaison fonctionnelle avec une séquence du contrôle de l'expression, qui n'est pas réprimable par une protéine répresseur de la tétracycline ;
(ii) on cultive la cellule de (i) dans un milieu approprié dans des conditions qui conduisent à l'expression du gène codant pour le peptide hétérologue, et
(iii) on isole le polypeptide hétérologue de la cellule ou du milieu.

22. Procédé selon la revendication 21, **caractérisé en ce que** l'on utilise une cellule, qui contient la séquence régulatrice du contrôle de l'expression et le gène de structure du répresseur de la tétracycline sur un seul vecteur.

23. Procédé selon la revendication 21, **caractérisé en ce que** l'on utilise une cellule, qui contient la séquence régulatrice du contrôle de l'expression et le gène de structure du répresseur de la tétracycline sur deux vecteurs différents, compatibles l'un avec l'autre.

24. Procédé selon l'une quelconque des revendications 21 à 23, **caractérisé en ce que** la culture de la cellule à l'étape (ii) est d'abord réalisée jusqu'à ce que l'on atteigne une densité cellulaire prédéterminée dans des conditions telles que l'expression du gène codant pour le peptide hétérologue est essentiellement réprimée, et qu'après avoir atteint la densité cellulaire prédéterminée, l'expression du gène est complètement ou partiellement induite.

25. Procédé selon la revendication 24, **caractérisé en ce que** l'induction est réalisée par addition de tétracycline ou d'un dérivé de la tétracycline.

26. Procédé selon la revendication 25, **caractérisé en ce que** l'induction est réalisée par addition d'anhydrotétracycline.

27. Procédé selon la revendication 24 ou 25, **caractérisé en ce que** l'addition de l'inducteur est réalisée jusqu'à une concentration finale allant de 5 à 500 µg/litre de milieu.

28. Procédé selon l'une quelconque des revendications 21 à 27, **caractérisé en ce que** l'on cultive les cellules dans un milieu minimal.
